# EUROPÄISCHE PATENTSCHRIFT

(11) **EP 2 885 353 B1**
(45) Veröffentlichungstag und Bekanntmachung des Hinweises auf die Patenterteilung: **02.03.2016**
(21) Anmeldenummer: 13747835.0
(22) Anmeldetag: 08.08.2013
(51) Int. Cl.: C08L 83/04, A61Q 5/00, C08K 5/49

(54) **VERFAHREN ZUR HERSTELLUNG VON SILICONEMULSIONEN**
METHOD FOR PRODUCING SILICONE EMULSIONS
PROCÉDÉ POUR PRODUIRE DES ÉMULSIONS DE SILICONE

(30) Priorität: 14.08.2012 DE 102012214429
(43) Veröffentlichungstag der Anmeldung: 24.06.2015
(73) Patentinhaber: Wacker Chemie AG, 81737 München (DE)
(72) Erfinder: RAUTSCHEK, Holger, 01612 Nünchritz (DE); KAUSCHKE, Marco, 01612 Glaubitz (DE)
(74) Vertreter: Budczinski, Angelika
(86) Internationale Anmeldenummer: PCT/EP2013/066598
(87) Internationale Veröffentlichungsnummer: WO 2014/026900

(56) Entgegenhaltungen:
- DE-A1-102011 002 668

## Beschreibung

Die Erfindung betrifft ein Verfahren zur Herstellung von wässrigen Siliconemulsionen, die hochviskose Polyorganosiloxane enthalten und einen besonders niedrigen Gehalt an cyclischen Siloxanen aufweisen, sowie deren Verwendung.

Silicone sind vielfältig einsetzbar. Um die Anwendung und Dosierung insbesondere bei viskosen Produkten zu erleichtern, ist es für viele Anwendungen wünschenswert, dass die siliziumorganischen Verbindungen in verdünnter Form vorliegen. Die Verwendung von organischen Lösemitteln, wie Benzol oder Chlorkohlenwasserstoffen, für diesen Zweck ist zwar möglich, jedoch aus ökologischer und arbeitsmedizinischer Sicht nachteilig. Deshalb erfolgt der Einsatz meist in Form von wässrigen Emulsionen oder Dispersionen, üblicherweise als Öl-in-Wasser-Emulsionen (O/W-Emulsionen), die mit Wasser verdünnbar sind. Als Ölphase werden dabei die mit Wasser nicht mischbaren siliziumorganischen Verbindungen verstanden, gegebenenfalls gelöst in organischen Lösemitteln.

Für viele Anwendungen ist es vorteilhaft, wenn das Silicon ein hohes Molekulargewicht und somit eine hohe Viskosität hat. Ein bekannter Weg zu Emulsionen zu gelangen, die ein hochmolekulares Silicon enthalten, ist die Emulsionspolymerisation von linearen Oligomeren mit endständigen Silanolgruppen. Aus diesen Oligomeren wird in Gegenwart von grenzflächenaktiven Kondensationskatalysatoren und einer sehr kleinen Wassermenge eine Paste gebildet, in der die Polykondensation stattfindet, bevor der eigentliche Emulgierprozess beendet ist. Anschließend wird der Emulgierprozess abgeschlossen, indem diese Paste auf die gewünschte Konzentration verdünnt wird (EP 93 310 B2). Dabei werden jedoch als unerwünschtes Nebenprodukt flüchtige cyclische Siloxane gebildet. Eine Verminderung des Anteils an diesen flüchtigen Siloxanen kann z.B. dadurch erfolgen, dass erst eine Emulsion aus der Salzform des anionischen Emulgators/ Katalysators hergestellt wird und diese dann durch Zugabe von Säure aktiviert wird (EP-A 1 072 629). Das erhöht letztlich den Salzanteil in der Emulsion, was für die Stabilität nachteilig ist.

Spezielle Emulgatoren auf der Basis von Taurocholaten tragen ebenfalls zur Reduzierung der Menge an Cyclen bei, die bei der Emulsionskondensation von Siloxanoligomeren gebildet werden (WO 2006 102 010). Aber auch hier werden, wie die Ausführungsbeispiele deutlich zeigen, über 1 % Octamethylcyclotetrasiloxan gebildet.

Eine deutliche Verringerung des Anteils an neu gebildeten cyclischen Siloxanen ist möglich, wenn als die Emulsionskondensation katalysierende Emulgatoren saure Alkylphosphate eingesetzt werden (DE 102 009 029 520 A1).

Den Verfahren nach dem Stand der Technik ist gemeinsam, dass der anionische Emulgator, der die Emulsionspolymerisation katalysiert, bereits bei der Emulsionsherstellung zugesetzt werden muss. Dass der Katalysator auch als Emulgator wirkt, wird nach dem Stand der Technik als Vorteil herausgestellt (DE-OS 1595535). Im Gegenteil, um die Emulsionspolymerisation nicht zu verzögern oder zu behindern wird vorgeschlagen, dass die Emulsion mit einer Sulfonsäure hergestellt wird und ein nichtionogener Emulgator erst nach Abschluss der Emulsionspolymerisation zugesetzt wird (US-A 3,706,695).

Auf der anderen Seite werden derartige Emulsionen praktisch oft in der Art und Weise hergestellt, dass entweder mehrere Chargen diskontinuierlich hergestellt und in einen Reifetank transferiert werden oder eine kontinuierliche Kampagne über einen bestimmten Zeitraum in einen Reifetank produziert wird, wo dann nach Erreichen der gewünschten Viskosität die Reaktion durch Neutralisation abgebrochen wird. Hinzu kommt, dass bei der Emulsionsherstellung durch die Homogenisierapparate mechanische Energie in die Emulsion eingetragen wird, was letztlich zu einer Temperatursteigerung und vermehrten Cyclenbildung führt. Dabei ist es unvermeidlich, dass ein nicht geringer Teil der Emulsion länger als erforderlich im Tank verweilt, wodurch der Anteil an cyclischen Oligomeren das tolerable Maß übersteigen kann. Deshalb besteht der Bedarf durch ein verbessertes Verfahren die gewünschten Produkteigenschaften insbesondere einen niedrigen Gehalt an cyclische Siloxanen mit größerer Sicherheit zu erreichen.

Gegenstand der Erfindung ist ein Verfahren zur Herstellung von Emulsionen von Organopolysiloxanen, dadurch gekennzeichnet, dass
in einem ersten Schritt
(a) Polyorganosiloxane enthaltend Einheiten der allgemeinen Formel

   R²ₐ(R¹O)_{b}SiO_{(4-a-b)/2} (I),

   worin
   R² gleich oder verschieden sein kann und einen einwertigen, gegebenenfalls substituierten Kohlenwasserstoffrest mit 1 bis 30 Kohlenstoffatomen oder Wasserstoffatom bedeutet,
   R¹ gleich oder verschieden sein kann und Wasserstoffatom oder einen einwertigen, gegebenenfalls substituierten Kohlenwasserstoffrest bedeutet,
   a 0, 1, 2 oder 3 ist und
   b 0, 1, 2 oder 3 ist,
   mit der Maßgabe, dass die Summe a+b kleiner oder gleich 3 ist und die Organopolysiloxane 5 bis 500 Einheiten der Formel (I) enthalten und in mindestens einer Einheit b verschieden 0 ist,
(b) nichtionogene Emulgatoren,
(c) Wasser und
   gegebenenfalls
(d) weitere Stoffe
   durch Rühren und/oder Homogenisieren vermischt werden,
   in einem zweiten Schritt
   zu der im ersten Schritt erhaltenen Emulsion
(e) saure oberflächenaktive Verbindungen der allgemeinen Formel

   (RO)ₙP(O)(OH)₍₃₋ₙ₎ (II),

   worin
   R gleich oder verschieden sein kann und einwertige Kohlenwasserstoffreste mit 4 bis 30 Kohlenstoffatomen bedeutet und n 1 oder 2 ist,
   bei einer Temperatur unter 30 °C, vorzugsweise bei unter 20 °C, insbesondere bei unter 10 °C, wozu die im ersten Schritt erhaltene Emulsion gegebenenfalls abgekühlt werden muss, zugegeben und die Polyorganosiloxane (a) kondensieren gelassen werden bis die gewünschte Viskosität erreicht ist und
   in einem dritten Schritt
   die im zweiten Schritt erhaltene Mischung mit Base (f) versetzt wird, so dass der pH-Wert der Emulsion größer als 5 ist, und gegebenenfalls weiteres Wasser (c) und/oder weitere Stoffe (d) zugegeben werden.

Im Rahmen der vorliegenden Erfindung wird der pH-Wert mit einer Elektrode entsprechend der US Pharmacopeia USP 33 bei 20 °C gemessen.

Als Misch- und Homogenisierwerkzeuge, die im erfindungsgemäßen Verfahren eingesetzt werden können, sind alle dem Fachmann bekannten Emulgiergeräte, wie beispielsweise schnelllaufende Rührer, Dissolverscheiben, Rotor-Stator-Homogenisatoren, Ultraschallhomogenisatoren und Hochdruckhomogenisatoren verschiedenster Bauart, geeignet. Wenn große Teilchen gewünscht sind, sind auch langsam laufende Rührer geeignet.

Das erfindungsgemäße Verfahren kann kontinuierlich, semikontinuierlich oder diskontinuierlich betrieben werden.

Bei den Polyorganosiloxanen (a), welche in dem erfindungsgemäßen Verfahren verwendet werden, handelt es sich vorzugsweise um solche enthaltend Einheiten der Formel (I), besonders bevorzugt um solche aus Einheiten der Formel (I) mit einem durchschnittlichen Wert von a von 1,90 bis 2,05 und einem durchschnittlichen Wert von b von 0,001 bis 0,2, insbesondere um solche aus Einheiten der Formel (I) mit R¹ gleich Wasserstoffatom, R² gleich Methylrest und einem durchschnittlichen Wert von a von 1,990 bis 2,005 und einem durchschnittlichen Wert von b von 0,01 bis 0,1. Ganz besonders bevorzugt handelt es sich bei den Polyorganosiloxanen (a) um Dimethylpolysiloxane, die Dimethylhydroxysiloxyendgruppen tragen.

Beispiele für Kohlenwasserstoffreste R² sind Alkylreste, wie der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neoPentyl-, tert.-Pentylrest; Hexylreste, wie der n-Hexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest; Dodecylreste, wie der n-Dodecylrest; Octadecylreste, wie der n-Octadecylrest; Cycloalkylreste, wie der Cyclopentyl-, Cyclohexyl-, Cycloheptylrest und Methylcyclohexylreste; Alkenylreste, wie der Vinyl-, 1-Propenyl- und der 2-Propenylrest; Arylreste, wie der Phenyl-, Naphthyl-, Anthryl- und Phenanthrylrest; Alkarylreste, wie o-, m-, p-Tolylreste; Xylylreste und Ethylphenylreste; und Aralkylreste, wie der Benzylrest, der der α- und der β-Phenylethylrest.

Beispiele für substituierte Reste R² sind mit Halogen-, Cyano-, Glycidoxy-, Polyalkylenglycol- oder Aminogruppen substituierte Reste, wie beispielsweise Trifluorpropyl-, Cyanoethyl-, Glycidoxypropyl-, Polyalkylenglycolpropyl-, Aminopropyl- oder Aminoethylaminopropylreste.

Bevorzugt hat in den Einheiten der Formel (I) maximal ein Rest R² die Bedeutung von Wasserstoffatom.

Bevorzugt handelt es sich bei Rest R² um Kohlenwasserstoffreste mit 1 bis 18 Kohlenstoffatomen, besonders bevorzugt um den Methyl- oder den Phenylrest, wobei insbesondere mehr als 80 Mol-% der Reste R² im Siloxan (a) die Bedeutung von Methylreste haben.

Beispiele für Reste R¹ sind die für Reste R² angegebenen Beispiele.

Bevorzugt handelt es sich bei Rest R¹ um Wasserstoffatom und Kohlenwasserstoffreste mit 1 bis 4 Kohlenstoffatomen, besonders bevorzugt um Wasserstoffatom.

In Formel (I) hat die Summe a+b einen Wert von bevorzugt durchschnittlich 1,5 bis 2,4, besonders bevorzugt durchschnittlich 1,9 bis 2,3, insbesondere durchschnittlich 1,95 bis 2,1.

Die im ersten Schritt des erfindungsgemäßen Verfahrens eingesetzten Siloxane (a) bestehen bevorzugt aus 5 bis 500, besonders bevorzugt aus 10 bis 200, insbesondere aus 20 bis 100, Einheiten der Formel (I).

In bevorzugt 0,4 bis 40 %, besonders bevorzugt 2 bis 10 %, der Einheiten der Formel (I) der im ersten Schritt des erfindungsgemäßen Verfahrens eingesetzten Siloxane (a) ist b ungleich 0.

Beispiele für erfindungsgemäß eingesetzte Siloxane (a) sind mit Alkoxy- oder Hydroxygruppen terminierte Polydiorganosiloxane, insbesondere Polydiethyl- und Polydimethylsiloxane.

Die im ersten Schritt des erfindungsgemäßen Verfahrens eingesetzten Siloxane (a) haben eine Viskosität von bevorzugt 5 bis 10 000 mm²/s, besonders bevorzugt 10 bis 500 mm²/s, insbesondere 30 bis 100 mm²/s, jeweils bei 25 °C.

Bevorzugt handelt es sich bei den Siloxanen (a) um solche der Formel

HO[SiR²₂O]_{c}-H (III),

wobei R² eine der obengenannten Bedeutungen hat, insbesondere Methylrest, und c einen Wert von 5 bis 500, bevorzugt von 10 bis 200, besonders bevorzugt aus 20 bis 100, besitzt.

Die erfindungsgemäß eingesetzten Polysiloxane (a) sind handelsübliche Produkte bzw. können nach bekannten Verfahren hergestellt werden.

Bei den im erfindungsgemäßen Verfahren eingesetzten nichtionogenen Emulgatoren kann es sich um beliebige, bisher bekannte Emulgatoren handeln.

Im ersten Schritt des erfindungsgemäßen Verfahrens werden Emulgatoren (b) in Mengen von bevorzugt 1 bis 40 Gewichtsteilen, besonders bevorzugt 5 bis 30 Gewichtsteilen, jeweils bezogen auf 100 Gewichtsteile Komponente (a) eingesetzt.

Bevorzugt werden als nichtionogene Emulgatoren (b) solche eingesetzt, die ausgewählt werden aus
(b1) ethoxylierten Triglyceriden mit 40 bis 400 Ethylenglycolgrupppen,
(b2) ethoxylierten Sorbitanestern von Fettsäuren mit 12 bis 18 Kohlenstoffatomen und 10 bis 40 Ethylenglycolgruppen,
(b3) Verbindungen der Formel

   R³-O- (CH₂CH₂O)ₘ-H (IV)

   und
(b4) Verbindungen der Formel

   R⁴CH₂C(O)-O-(CH₂CH₂O)ₚ-H (V),

   worin
   R³ einen Alkylrest mit 10 bis 30 Kohlenstoffatomen bedeutet,
   R⁴ einen Alkylrest mit 10 bis 30 Kohlenstoffatomen bedeutet,
   m einen Wert von 5 bis 100 darstellt und
   p einen Wert von 5 bis 100 annimmt.

Beispiele für Reste R³ und R⁴ sind unabhängig voneinander der n-Decyl-, n-Dodecyl-, n-Tetradecyl-, n-Hexadecyl-, n-Octadecyl- und der n-Octadecenylrest.

Bevorzugt handelt es sich bei Rest R³ und R⁴ unabhängig voneinander um Alkylreste mit 12 bis 20 Kohlenstoffatomen, besonders bevorzugt um lineare Alkylreste.

Insbesondere handelt es sich bei Rest R³ und R⁴ unabhängig voneinander um lineare Alkylreste mit 12 bis 20 Kohlenstoffatomen und einer geraden Anzahl an Kohlenstoffatomen.

Bevorzugt hat m einen Wert von 10 bis 40, besonders bevorzugt von 20 bis 40.

Bevorzugt hat p einen Wert von 10 bis 50, besonders bevorzugt von 20 bis 50.

Beispiele für ethoxylierte Triglyceride mit 40 bis 400 Ethylenglycolgrupppen (b1) sind ethoxyliertes Rizinusöl mit 200 Ethylenglycoleinheiten, ethoxyliertes Rizinusöl mit 40 Ethylenglycoleinheiten und ethoxyliertes hydriertes Rizinusöl mit 200 Ethylenglycoleinheiten.

Beispiele für ethoxylierte Sorbitanester von Fettsäuren mit 12 bis 18 Kohlenstoffatomen und 10 bis 40 Ethylenglycolgruppen (b2) sind Polyoxyethylen(20)Sorbitanstearat (Polysorbat 60), Polyoxyethylen(20)Sorbitantristearat (Polysorbat 65), Polyoxyethylen(20)Sorbitanoleat (Polysorbat 80) und Polyoxyethylen(20)Sorbitanlaurat (Polysorbat 20).

Beispiele für Verbindungen (b3) der Formel (IV) sind
i-C₁₃H₂₇-O- (CH₂CH₂O)₁₀-H,
C₁₈H₃₇-O-(CH₂CH₂O)₂₀-H,
C₁₈H₃₅-O-(CH₂CH₂O)₂₀-H und
C₁₂H₂₃-O-(CH₂CH₂O)₂₃-H.

Beispiele für Verbindungen (b4) der Formel (V) sind
C₁₆H₃₃-CH₂-C(O)-O-(CH₂CH₂O)₂₀-H, C₁₆H₃₃-CH₂-C(O)-O-(CH₂CH₂O)₃₀-H,
C₁₆H₃₃-CH₂-C(O)-O-(CH₂CH₂O)₄₀-H und C₁₆H₃₃-CH₂-C(O)-O-(CH₂CH₂₀)₁₀₀-H.

Vorzugsweise hat der in der erfindungsgemäßen Emulsion enthaltene Emulgator (b) einen HLB-Wert größer 14, besonders bevorzugt größer 15,5, insbesondere von 16,5 bis 20. Der HLB-Wert ist Ausdruck des Gleichgewichts zwischen hydrophilen und hydrophoben Gruppen eines Emulgators. Die Definition des HLB-Werts sowie Verfahren zu deren Ermittlung sind dem Fachmann bekannt und z.B. in Journal of Colloid and Interface Science 298 (2006) 441-450 sowie der dort zitierten Literatur insbesondere Zitat [23] beschrieben.

Vorzugsweise handelt es sich bei Emulgator (b) um eine Verbindung der Formel (IV).

Beispiele für Reste R in Verbindung der Formel (II) sind verzweigte oder unverzweigte Alkylreste, wie Butyl-, Hexyl-, 2-Ethylhexyl-, Octyl-, Isononyl-, n-Decyl-, Dodecyl-, Isotridecyl- und n-Tetradecylreste, ungesättigte aliphatische Reste, wie Oleylreste, sowie aromatische Reste, wie Phenyl-, Toloyl-, Xylyl-, Nonylphenyl-, Naphthyl-, Anthracyl-, Tristyrylphenyl-oder Benzylreste.

Bevorzugt handelt es sich bei Rest R um Alkylreste mit 4 bis 18 Kohlenstoffatomen, besonders bevorzugt um n-Butyl-, n-Octyl-, 2-Ethylhexyl-, n-Decyl-, n-Dodecyl- oder n-Tetradecylreste, insbesondere um n-Octyl- und n-Decylreste.

Beispiele für erfindungsgemäß eingesetzte Verbindungen der Formel (II) sind Di-n-butylphosphat, Di-n-hexylphosphat, Mono-n-octylphosphat, Di-n-octylphosphat, Mono-2-ethylhexylphosphat, Di-2-ethylhexylphosphat, Mono-i-nonylphosphat, Di-i-nonylphosphat, Mono-n-decylphosphat, n-Octyl-n-Decylphosphat, Di-n-decylphosphat, Monoisotridecylphosphat, Di-n-nonylphenylphosphat, Monooleylphosphat und Distearylphosphat.

Bevorzugt handelt es sich bei den erfindungsgemäß eingesetzten Verbindungen der Formel (II) um Mono-n-octylphosphat, Di-n-octylphosphat, Mono-n-decylphosphat, n-Octyl-n-Decylphosphat und Di-n-decylphosphat.

Vorzugsweise handelt es sich bei den erfindungsgemäß eingesetzten Verbindungen der Formel (II) um Mischungen von Diestern und Monoestern.

Herstellungsbedingt können die als Komponenten (e) eingesetzten Verbindungen sogenannte Pyrophosphate, Polyphosphate oder Diphosphate, die nicht der Formel (II) entsprechen, enthalten. Bei diesen Verbindungen sind zwei oder mehr Verbindungen der Formel (I) durch P-O-P Brücken miteinander verknüpft. In der erfindungsgemäß eingesetzten Komponente (e) können bis zu 50 % der Phosphoratome in dieser Form vorliegen, vorzugsweise liegen weniger als 10 % der Phosphoratome als Pyrophosphate vor, insbesondere weniger als 2 %.

Die Säurezahl der erfindungsgemäß eingesetzten Verbindung der Formel (II) liegt vorzugsweise im Bereich von 100 bis 500, besonders bevorzugt im Bereich von 200 bis 400.

Die Säurezahl der in dem erfindungsgemäßen Verfahren verwendeten Verbindungen der Formel (II) wird durch deren Anzahl an freien OH-Gruppen sowie deren Molmasse bestimmt, also die Menge an KOH in mg, die zur Neutralisation von 1 g Verbindung der Formel (II) benötigt wird.

Als Wasser (c) können alle Arten von Wässern eingesetzt werden, die auch bisher zur Herstellung von Dispersionen eingesetzt wurden.

Als Wasser (c) wird vorzugsweise teil- oder vollentsalztes Wasser, destilliertes oder (mehrfach) redestilliertes Wasser, Wässer für medizinische oder pharmazeutische Zwecke, wie z.B. gereinigtes Wasser (Aqua purificata gemäß Pharm. Eur.) eingesetzt.

Das erfindungsgemäß eingesetzte Wasser (c) hat vorzugsweise eine Leitfähigkeit von weniger als 50 µS/cm, besonders bevorzugt weniger als 10 µS/cm, insbesondere weniger als 1,3 µS/cm, jeweils bei 25 °C und 1010 hPa.

In dem erfindungsgemäßen Verfahren werden vorzugsweise insgesamt 50 bis 1000 Gewichtsteile Wasser, bezogen auf 100 Gewichtsteile Komponenten (a), eingesetzt.

Zusätzlich zu den Komponenten (a), (b), (c) (e) und (f) können im erfindungsgemäßen Verfahren alle weiteren Stoffe (d), die üblicherweise Siliconemulsionen zugesetzt werden und verschieden sind zu den Komponenten (a), (b), (c) (e) und (f), eingesetzt werden, wie z.B. weitere Siloxane, Silane insbesondere Alkoxysilane, weitere Emulgatoren, Verdicker und/oder Schutzkolloide sowie Additive, wie beispielsweise Konservierungsmittel, Desinfektionsmittel, Netzmittel, Korrosionsinhibitoren, Farbstoffe und Duftstoffe. Der Zusatz dieser Komponenten kann jedoch auch nach einem späteren Verfahrensschritt, z.B. nach dem 3. Schritt, erfolgen.

Beispiele für weitere Siloxane (d), die erfindungsgemäß eingesetzt werden können, sind solche der Formel (I) mit b gleich 0, wie z.B. trimethylsiloxyterminierte Polydimethylsiloxane. Solche Siloxane (d) werden vorteilhafterweise eingesetzt, um die nach der Kondensationsreaktion erhaltenen Viskosität des Polysiloxans in der Emulsion zu steuern.

Falls weitere Siloxane (d) eingesetzt werden, handelt es sich um Mengen von bevorzugt 0,01 bis 10 Gewichtsteilen, bezogen auf 100 Gewichtsteile Komponente (a). Im erfindungsgemäßen Verfahren werden bevorzugt keine weiteren Siloxane (d) eingesetzt.

Beispiele für weitere Silane (d), die erfindungsgemäß eingesetzt werden können, sind Methyltrimethoxysilan, Tetraethoxysilan, Vinyltriethoxysilan oder deren Hydrolyse-/Kondensations-produkte. Solche Silane (d) werden vorteilhafterweise eingesetzt, um verzweigte oder vernetzte Siloxane z.B. solche, die nach der Trocknung der Emulsion elastische Filme bilden, zu erhalten. Diese Silane (d) können auch nach dem dritten Schritt zugesetzt werden.

Falls weitere Silane (d) eingesetzt werden, handelt es sich um Mengen von bevorzugt 0,01 bis 10 Gewichtsteilen, bezogen auf 100 Gewichtsteile Komponente (a).

Im erfindungsgemäßen Verfahren werden vorzugsweise ausschließlich Siloxane enthaltend Einheiten der Formel (I), insbesondere Siloxane der Formel (III), und keine weiteren Siloxane oder Silane eingesetzt.

Beispiele für weitere Emulgatoren (d), die im erfindungsgemäßen Verfahren eingesetzt werden können, sind andere dem Fachmann bekannte Emulgatoren, die verschieden sind zu Komponenten (b) und (e), insbesondere Alkali- oder Aminsalze von Verbindungen der Formel (II).

Es ist auch möglich, aber nicht bevorzugt, einen Teil der im erfindungsgemäßen Verfahren eingesetzten Komponente (e) bereits im ersten Schritt zuzusetzen, mit der Maßgabe, dass der pH-Wert der im ersten Schritt erhaltenen Emulsion größer als 3,5 ist.

Falls bei dem erfindungsgemäßen Verfahren als Komponente (d) Verdicker bzw. Schutzkolloide eingesetzt werden, handelt es sich bevorzugt um Acrylsäurecopolymere.

Falls Verdicker und/oder Schutzkolloide (d) eingesetzt werden, handelt es sich um Mengen von bevorzugt 0,01 bis 2 Gewichtsteilen, bezogen auf 100 Gewichtsteile Komponente (a). Im erfindungsgemäßen Verfahren wird bevorzugt kein Verdicker und/oder Schutzkolloid (d) eingesetzt.

Beispiele für Additive (d), die erfindungsgemäß eingesetzt werden können, sind z.B. dem Fachmann bekannte Konservierungsmittel, Farb- oder Duftstoffe, insbesondere Konservierungsmittel, wie Methylisothiazolinon, Chlormethylisothiazolinon, Benzylisothiazolinon, Phenoxyethanol, Methylparaben, Ethylparaben, Propylparaben, Butylparaben, Isobutylparaben, Alkalibenzoate, Alkalisorbate, Iodopropynylbutylcarbamat, Benzylalkohol und 2-Brom-2-nitropropan-1,3-diol.

Falls Additive (d) eingesetzt werden, handelt es sich um Mengen von bevorzugt 0,0005 bis 2 Gewichtsteilen, bezogen auf 100 Gewichtsteile Komponente (a). Im erfindungsgemäßen Verfahren werden bevorzugt Additive (d), insbesondere Konservierungsmittel, eingesetzt. Konservierungsmittel (d) werden bevorzugt im dritten Schritt zugesetzt und nicht im ersten Schritt.

Im ersten Schritt des erfindungsgemäßen Verfahrens können alle Komponenten durch Rühren und/oder Homogenisieren, z.B. in beliebiger Reihenfolge, miteinander vermischt werden, wobei die Umfangsgeschwindigkeit des Rührers und/oder Rotor-Stator Homogenisators bevorzugt größer als 5 m/s, besonders bevorzugt größer als 10 m/s ist, insbesondere 5 bis 50 m/s.

Der pH-Wert der im ersten Schritt des erfindungsgemäßen Verfahrens erhaltenen Emulsion beträgt vorzugsweise mehr als 3,5 insbesondere mehr als 4,5. Vorzugsweise ist der pH Wert der im ersten Schritt erhaltenen Emulsion jedoch nicht größer als 9, insbesondere nicht größer als 8.

Vorzugsweise wird die im ersten Schritt hergestellte Emulsion enthaltend Komponenten (a), (b), (c) und gegebenenfalls (d) und (e) so hergestellt, dass erst ein Teil der Komponente (c) mit den übrigen Komponenten vermischt wird und eine hochviskos und nicht fließfähige Paste entsteht. Besonders bevorzugt ist es, wenn die Fließgrenze (entsprechend DIN 53019-1 und dort zitierten Normen) dieser pastösen Vormischung größer als 100 Pa, insbesondere größer als 1000 Pa, ist. Vorzugsweise wird diese pastöse Vormischung dann unter Einwirkung von Scherenergie homogenisiert bis die gewünschte Teilchengröße erreicht wird und mit Wasser (c) unter Rühren und/oder Homogenisieren verdünnt, so dass eine fließfähige Emulsion gebildet wird, die vorzugsweise mehr als 50 Gewichtsteile, besonders bevorzugt 50 bis 1000 Gewichtsteile, insbesondere 80 bis 500 Gewichtsteile, Wasser, jeweils bezogen auf 100 Gewichtsteile Komponente (a) enthält.

Im ersten Schritt des erfindungsgemäßen Verfahrens werden über die Komponenten (a), (b) und (c) sowie gegebenenfalls (d) und (e) hinausgehend vorzugsweise keine weiteren Komponenten eingesetzt.

Der erste Schritt des erfindungsgemäßen Verfahrens wird bei Temperaturen von vorzugsweise 5 bis 80 °C, insbesondere 10 bis 50 °C, und dem Druck der umgebenden Atmosphäre, also zwischen 900 und 1100 hPa, durchgeführt. Der erste Schritt kann aber auch bei einem erhöhten Druck von bis zu 20000 hPa, vorzugsweise von bis zu 10000 hPa, durchgeführt werden.

Die im ersten Schritt des erfindungsgemäßen Verfahrens erhaltene Mischung hat eine Partikelgröße (Medianwert der Volumenverteilung) von bevorzugt kleiner als 1 µm, besonders bevorzugt kleiner als 500 nm, insbesondere 50 bis 200 nm. Die Partikelgröße kann mit Laserstreuung oder durch Laserbeugung nach DIN ISO 13320 bestimmt werden.

In einer speziellen Variante des erfindungsgemäßen Verfahrens werden Partikelgrößen (Mittelwert der Volumenverteilung) größer >1 µm angestrebt, insbesondere Partikelgrößen von 5 bis 50 µm wobei dann das Verfahren vorzugsweise durch scherkraftarmes Mischen, z.B. einfaches Rühren der Komponenten, ausgeübt wird.

Die für die Durchführung des ersten Schritts des erfindungsgemäßen Verfahrens benötigte Zeit beträgt vorzugsweise 5 Minuten bis 4 Stunden, besonders bevorzugt 30 Minuten bis 3 Stunden, insbesondere 1 bis 2 Stunden.

Im zweiten Schritt des erfindungsgemäßen Verfahrens wird die im ersten Schritt hergestellte Mischung bei einer Temperatur von unter 30 °C mit der Komponente (e) vermischt, wobei die Temperaturuntergrenze bevorzugt der Schmelzpunkt der Mischung bildet.

Falls die Temperatur der im ersten Schritt erhaltenen Emulsion höher als die im zweiten Schritt geforderte Temperatur von unterhalb von 30 °C ist, wird die Emulsion vor Zugabe der Komponente (e) im zweiten Schritt auf die gewünschte Temperatur abgekühlt. Dieses gegebenenfalls durchgeführte Abkühlen erfolgt bevorzugt durch Kühlung über die Behälterwand oder einen Wärmetauscher, wobei als Kühlmedium alle bekannten Wärmeträger eingesetzt werden können z.B. Wasser, Kühlsole oder organische Wärmeträger wie Marlotherm^{®}.

Der Zusatz der Komponente (e) erfolgt durch einfaches Rühren, ein Homogenisieren ist möglich aber meist nicht erforderlich und deshalb nicht bevorzugt. Um die Dosierung zu erleichtern ist es möglich, aber nicht bevorzugt, Komponente (e) in Wasser (c) zu dispergieren.

Der pH-Wert der im zweiten Schritt des erfindungsgemäßen Verfahrens erhaltenen Emulsion beträgt vorzugsweise weniger als 2,5 besonders bevorzugt weniger als 2,0, insbesondere 1,4 bis 1,8.

Im erfindungsgemäßen Verfahren wird die Komponente (e) in Mengen von bevorzugt 1 bis 40 Gewichtsteilen, besonders bevorzugt 2 bis 20 Gewichtsteilen, insbesondere von 4 bis 10 Gewichtsteilen, jeweils bezogen auf 100 Gewichtsteile Polysiloxan (a) zugesetzt. Die Einsatzmenge kann auch geteilt werden, so dass vorzugsweise bis zu maximal 40 Gew.-% der gesamten Einsatzmenge von (e) bereits im ersten Schritt und der verbleibende Teil im zweiten Schritt des erfindungsgemäßen Verfahrens zugesetzt wird. Das kann vorteilhaft sein, um eine kleine Teilchengröße mit niedrigen Cyclengehalten zu verbinden, wenn die im ersten Schritt zugesetzte Teilmenge zur Emulsionsbildung beiträgt, aber in dieser Phase des Verfahrens nur in keinem oder geringem Maße Reaktionen am Siloxan katalysiert. Beim erfindungsgemäßen Verfahren wird die Gesamtmenge an eingesetzter Komponente (e) vorzugsweise im zweiten Schritt eingesetzt.

Im zweiten Schritt des erfindungsgemäßen Verfahrens werden über die Komponenten (e) und gegebenenfalls (c) hinausgehend vorzugsweise keine weiteren Komponenten eingesetzt.

Anschließend erfolgt die Polykondensation der Organopolysiloxane (a) bis die Viskosität erreicht ist, wie sie in der erfindungsgemäßen Emulsion gewünscht wird, also eine Viskosität von bevorzugt größer als 10 000 mm²/s, besonders bevorzugt größer als 100 000 mm²/s, insbesondere größer als 1 000 000 mm²/s, jeweils bei 25 °C, gemessen nach DIN 53019.

Bevorzugt beträgt die Dauer der Kondensationsreaktion nach Zugabe von (e) im zweiten Schritt 1 bis 200 Stunden, besonders bevorzugt 8 bis 96 Stunden, insbesondere 12 bis 72 Stunden.

Der zweite Schritt des erfindungsgemäßen Verfahrens kann in dem gleichen Behälter erfolgen wie der erste Schritt. Die Emulsion kann aber auch in einen speziellen Behälter überführt werden, wo gegebenenfalls mehrere hintereinander hergestellte Ansätze für den zweiten Schritt vermischt werden. Es ist jedoch auch möglich den ersten Schritt kontinuierlich und den zweiten Schritt in einem Reifetank durchzuführen.

Die bei dem erfindungsgemäßen Verfahren gegebenenfalls als Kondensationsnebenprodukte anfallenden Alkohole, z.B. wenn R¹ in Formel (I) verschieden Wasserstoffatom ist, können in der Emulsion verbleiben oder auch entfernt werden, beispielsweise durch Destillation unter Vakuum oder durch Extraktion.

Der Druck ist bei der Durchführung dieses zweiten Verfahrensschrittes für die Produktqualität nicht wesentlich. Praktischerweise wird dieser Schritt z.B. beim Druck der umgebenden Atmosphäre, also zwischen 900 und 1100 hPa, durchgeführt, er kann auch bei einem erhöhten Druck von z.B. bis zu 20000 hPa, insbesondere von bis zu 10000 hPa, oder bei vermindertem Druck durchgeführt werden.

Beispiele für die im dritten Schritt des erfindungsgemäßen Verfahrens eingesetzten Basen (f) sind Alkalihydroxyde, wie NaOH und KOH, sowie Amine, wie z.B. Monoethanolamin und Triethanolamin, und Salze schwacher Säuren, wie z.B. Natriumcitrat, Natriumsilikat, Kaliumacetat oder Kaliumphosphat.

Bevorzugt handelt es sich bei den Basen (f), die im dritten Schritt des erfindungsgemäßen Verfahrens eingesetzt werden, um Alkali- bzw. Erdalkalihydroxide, Ammoniak und Amine, besonders bevorzugt um NaOH, KOH, Monoethanolamin und Triethanolamin.

Der pH-Wert der Emulsion nach Durchführung des erfindungsgemäßen dritten Schritts beträgt vorzugsweise 5 bis 10, besonders bevorzugt 6 bis 8, insbesondere in etwa 7.

Der dritte Schritt des erfindungsgemäßen Verfahrens wird bei Temperaturen von vorzugsweise 2 bis 30 °C, besonders bevorzugt 5 bis 20 °C, und einem Druck der umgebenden Atmosphäre, also zwischen 900 und 1100 hPa, durchgeführt.

Die erfindungsgemäß erhaltenen Emulsionen können nun gegebenenfalls beliebig mit weiterem Wasser (c) und/oder weiteren Stoffen (d), insbesondere Konservierungsmittel, vermischt werden.

Bevorzugt werden zusätzlich zu den Komponenten (a), (b), (c), (e), (f) und gegebenenfalls (d) im erfindungsgemäßen Verfahren keine weiteren Komponenten eingesetzt.

Bei den im erfindungsgemäßen Verfahren eingesetzten Komponenten kann es sich jeweils um eine Art einer solchen Komponente wie auch um ein Gemisch aus mindestens zwei Arten einer jeweiligen Komponente handeln.

Die erfindungsgemäß hergestellten Emulsionen enthalten vorteilhafterweise keinen bzw. nur einen sehr geringen Anteil an cyclischen Siloxanen, insbesondere an Octaorganylcyclotetrasiloxanen (D₄). Die Organylgruppen in den Cyclosiloxanen richten sich nach den Organylgruppen im eingesetzten Organopolysiloxan und sind bevorzugt Methylgruppen.

Die erfindungsgemäß hergestellten Emulsionen enthalten bevorzugt weniger als 0,2 Gew.-%, besonders bevorzugt weniger als 0,1 Gew.-%, Octaorganylcyclotetrasiloxan, insbesondere Octamethylcyclotetrasiloxan (D₄), jeweils bezogen auf eingesetzte Komponente (a).

Die erfindungsgemäß hergestellten Emulsionen haben einen Teilchendurchmesser von vorzugsweise 50 bis 1000 nm, besonders bevorzugt von 50 bis 500 nm, insbesondere von 50 bis 200 nm, wobei sich diese Angaben auf den Medianwert der Volumenverteilung gemessen nach dem Prinzip Laserstreuung oder der Fraunhoferbeugung (entsprechend ISO 13320) beziehen.

Die erfindungsgemäß hergestellten Emulsionen haben einen Gehalt an nichtflüchtigen Anteilen gemessen nach DIN EN ISO 3251 von bevorzugt 1 bis 80 Gew.-%, besonders bevorzugt von 10 bis 65 Gew.-%, insbesondere von 30 bis 60 Gew.-%.

Überraschenderweise wurde gefunden, dass es möglich ist, die sauren Verbindungen der Formel (II), welche die Polykondensation der Siloxane (a) katalysieren sollen, erst nach der Emulsionsherstellung im ersten Schritt, die z.B. ausschließlich mit nichtionogenen Emulgatoren erfolgen kann, zuzusetzen, ohne dass die Geschwindigkeit der Polykondensationsreaktion beeinträchtigt wird.

Die erfindungsgemäßen bzw. erfindungsgemäß hergestellten Emulsionen haben den Vorteil, dass sie hochviskose Polydiorganosiloxane enthalten und einen besonders niedrigen Gehalt an Cyclen aufweisen.

Des Weiteren haben die erfindungsgemäß hergestellten Emulsionen den Vorteil, dass sie sehr stabil und damit lange haltbar sind.

Die erfindungsgemäß hergestellten Emulsionen haben den Vorteil, dass sie lagerstabil sind und ausgezeichnete anwendungstechnische Eigenschaften besitzen, wie z.B. eine sehr gute Wirkung als Trenn- und Gleitmittel, eine gute Benetzungsfähigkeit auf unterschiedlichen Substraten, eine gute Konditionierwirkung in Haarpflegeprodukten, d.h. deutliche Reduzierung der Nass- und Trockenkämmkraft.

Das erfindungsgemäße Verfahren hat den Vorteil, dass auf einfache und kostengünstige Art Emulsionen mit hochmolekularen Siloxanen hergestellt werden können.

Das erfindungsgemäße Verfahren hat ferner den Vorteil, dass auch nach längerer Dauer des dritten Schritts der Anteil an cyclischen Siloxanen sehr gering bleibt, was z.B. bei einer kontinuierlichen Produktion mit einem breiteren Verweilzeitbereich besonders günstig ist.

Das erfindungsgemäße Verfahren hat den Vorteil, dass die Viskosität des Öles in einem weiten Bereich variiert und einfach eingestellt werden kann, ohne dass ein erhöhter Anteil an cyclischen Siloxanen gebildet wird.

Die erfindungsgemäß hergestellten Emulsionen sind für alle Zwecke einsetzbar, für die auch bisher Emulsionen mit hochviskosen Siloxanen eingesetzt worden sind, wie beispielsweise als Trennmittel, Gleitmittel, Hydrophobiermittel und zur Textilimprägnierung, bei der Verarbeitung von Gummi und Kunststoffen oder bei der Metallverarbeitung, für Glas und mineralische Baustoffe oder als Bestandteil von Körperpflegeprodukten.

In den nachfolgenden Beispielen beziehen sich alle Angaben von Teilen und Prozentsätzen, soweit nicht anders angegeben, auf das Gewicht. Sofern nicht anders angegeben, werden die folgenden Beispiele bei einem Druck der umgebenden Atmosphäre, also bei etwa 1010 hPa, und bei Raumtemperatur, also etwa 25 °C bzw. einer Temperatur, die sich beim Zusammengeben der Reaktanten bei Raumtemperatur ohne zusätzliche Heizung oder Kühlung einstellt, durchgeführt. Alle in den Beispielen angeführten Viskositätsangaben beziehen sich auf eine Temperatur von 25 °C.

Die in den nachfolgenden Beispielen hergestellten Emulsionen wurden wie folgt geprüft:
Die Teilchengröße wurde an mit einem Partikelgrößenanalysator Zetasizer ZEN1600/ Nano-S, Fa. Malvern, Software Version 6.01 mittels dynamischer Lichtstreuung bestimmt. Dazu wurden die Emulsionen mit gefiltertem und entgastem Wasser auf 0,5 % verdünnt.
Bei den grobteiligen Emulsionen wurde die Partikelgrößenmessung mit einem Malvern Mastersizer X (Malvern Instruments GmbH D-Herrenberg; Messprinzip: Fraunhofer Beugung entsprechend ISO 13320) durchgeführt.

Die angegebenen Werte für die Teilchengröße beziehen sich immer auf den Medianwert der Volumenverteilung D(50).

Die Messung-des pH-Wertes erfolgte entsprechend der US Pharmacopeia USP 33 bei 20 °C.

Zur Bestimmung der Ölviskosität wurden 20 g Emulsion mit 30 g Aceton versetzt, wobei sich die Emulsion trennte. Die Aceton/ Wasserphase wurde abgetrennt und der Vorgang noch einmal wiederholt. Anschließend wurde das Polymer dreimal mit Wasser gewaschen und bei 110 °C unter Rühren getrocknet, bis keine Wassertröpfchen mehr zu sehen waren und anschließend noch 8 Stunden bei 110 °C im Trockenschrank nachbehandelt. Die Viskosität wurde nach DIN 53019 mit einem Kegel-Platte-Viskosimeter MCR 300 (Paar-Physika) bei 25 °C und einem Schergefälle von 1/s bestimmt.

Zur Bestimmung des Gehaltes an Octamethylcyclotetrasiloxan (D₄) wurden 0,5 g der Probe mit 10 ml reinstem Aceton, das 120 ppm n-Dodecan als internen Standard enthielt, gemischt. Nach Schütten für eine Dauer von 16 Stunden bildeten sich zwei Phasen. Von der überstehenden klaren Phase wurden 10 µl in einen Gaschromatographen eingespritzt. Es wurde eine Doppelbestimmung durchgeführt. Der Gehalt an Octamethylcyclotetrasiloxan (D₄) in der Emulsion wurde anhand einer vorher aufgenommenen Kalibrierkurve bestimmt. Daraus konnte dann der D₄-Gehalt bezogen auf das Polysiloxan (a) berechnet werden.

Die Prüfergebnisse der nachfolgend aufgeführten Beispiele sind in Tabelle 1 zusammengefasst.

### Beispiel 1

100 Teile eines mit α,ω-hydroxyterminierten Polydimethylsiloxans mit einer Viskosität von 60 mPas und einer Temperatur von 20 °C wurden in einem Becher vorgelegt. Mit einem Rotor-Stator-Homogenisator (Ultra-Turrax, Umfangsgeschwindigkeit 16 m/s) wurden 14 Teile eines ethoxylierten Laurylalkohols der Formel C₁₂H₂₃-O-(CH_{Z}CH₂O)₂₃-H (erhältlich unter dem Namen "Brij 35" bei der Croda GmbH, D-Nettetal) und 10 Teile Wasser zugegeben und 10 min homogenisiert. Die entstandene gelartige Paste (Fließgrenze 3210 Pa) mit einer Partikelgröße von kleiner als 200 nm hatte eine Temperatur von 48°C. Diese Paste wurde innerhalb von 10 min mit 100 Teilen Wasser verdünnt und auf 5 °C abgekühlt. Der pH-Wert betrug 5,5.
Anschließend wurden 6 Teile eines Octyl-/Decylphosphats mit einer Säurezahl von 295 mg KOH/g, d.h. das in etwa aus gleichen Anteilen Monoester und Diester besteht (erhältlich unter dem Namen "Crodafos 810 A" bei der Croda GmbH, D-Nettetal), eingerührt. Der pH-Wert betrug 1,9. Die Emulsion wurde 48 Stunden bei 5 °C gelagert und anschließend mit Triethanolamin auf einen pH-Wert von 7 eingestellt, und es wurden 0,18 Teile Konservierungsmittel auf der Basis von Methylisothiazolinon in Kombination mit Ethylhexylglycerin (erhältlich unter der Bezeichnung "Euxyl K220" bei Schülke&Mayr GmbH, Norderstedt) zugesetzt.

### Beispiel 2

100 Teile eines mit α,ω-hydroxyterminierten Polydimethylsiloxans mit einer Viskosität von 60 mPas und einer Temperatur von 20 °C wurden in einem Becher vorgelegt. Mit einem Rotor-Stator-Homogenisator (Ultra-Turrax, Umfangsgeschwindigkeit 16 m/s) wurden 22 Teile eines ethoxylierten Laurylalkohols der Formel C₁₂H₂₃-O-(CH₂CH₂O)₂₃-H (erhältlich unter dem Namen "Brij 35" bei der Croda GmbH, D-Nettetal) und 10 Teile Wasser zugegeben und 10 min homogenisiert. Die entstandene gelartige Paste (Fließgrenze 2840 Pa) mit einer Partikelgröße von kleiner als 200 nm hatte eine Temperatur von 42 °C. Die Paste wurde innerhalb von 10 min mit 100 Teilen Wasser verdünnt und auf 5 °C abgekühlt. Der pH-Wert betrug 5,5.
Anschließend wurden 10 Teile eines Octyl-/Decylphosphats mit einer Säurezahl von 295 mg KOH/g (erhältlich unter dem Namen "Crodafos 810 A" bei der Croda GmbH, D-Nettetal) eingerührt. Der pH-Wert betrug 1,5. Die Emulsion wurde 24 Stunden bei 5 °C gelagert und anschließend mit Triethanolamin auf einen pH-Wert von 7 eingestellt, und es wurden 0,18 Teile Konservierungsmittel auf der Basis von Isothiazolinonen (erhältlich unter der Bezeichnung "Kathon CG" bei Acima Chemical Industries Ltd. CH-9471 Buchs/SG) zugesetzt.

### Beispiel 3

100 Teile eines mit α,ω-hydroxyterminierten Polydimethylsiloxans mit einer Viskosität von 60 mPas und einer Temperatur con 20 °C wurden in einem Becher vorgelegt. Mit einem Rotor-Stator-Homogenisator (Ultra-Turrax, Umfangsgeschwindigkeit 16 m/s) wurden 14 Teile eines ethoxylierten Laurylalkohols der Formel C₁₂H₂₃-O-(CH₂CH₂O)₂₃-H (erhältlich unter dem Namen "Brij 35" bei der Croda GmbH, D-Nettetal), 5 Teile eines Octyl-/Decylphosphats mit einer Säurezahl von 295 mg KOH/g (erhältlich unter dem Namen "Crodafos 810 A" bei der Croda GmbH, D-Nettetal), 1,7 Teile Triethanolamin und 10 Teile Wasser zugegeben und 10 min homogenisiert. Die entstehende gelartige Paste (Fließgrenze 1580 Pa) mit einer Partikelgröße von kleiner als 200 nm hatte eine Temperatur von 40 °C. Die Paste wurde innerhalb von 10 min mit 100 Teilen Wasser verdünnt und auf 5 °C abgekühlt. Die Emulsion hatte einen pH-Wert von 3,7.
Anschließend wurden weitere 5 Teile eines Octyl-/Decylphosphats mit einer Säurezahl von 295 mg KOH/g (erhältlich unter dem Namen "Crodafos 810 A" bei der Croda GmbH, D-Nettetal) eingerührt. Der pH-Wert betrug 2,3. Die Emulsion wurde 48 Stunden bei 5 °C gelagert und anschließend mit Triethanolamin auf einen pH-Wert von 7 eingestellt und es wurden 0,18 Teile Konservierungsmittel auf der Basis von Methylisothiazolinon in Kombination mit Ethylhexylglycerin (erhältlich unter der Bezeichnung "Euxyl K220" bei Schülke&Mayr GmbH, Norderstedt) zugesetzt.

### Beispiel 4

Die in Beispiel 2 beschriebene Arbeitsweise wurde wiederholt mit der Abänderung, dass anstelle von Octyl-/Decylphosphat 2-Ethylhexylphosphat mit einer Säurezahl von 300 mg KOH/g, d.h. das in etwa aus gleichen Anteilen Monoester und Diester besteht, eingesetzt wurde, das unter dem Namen "Servoxyl VPTZ 100" bei der Elementis GmbH, D-Köln erhältlich ist.

### Beispiel 5

Die in Beispiel 2 beschriebene Arbeitsweise wurde wiederholt mit der Abänderung, dass anstelle von Octyl-/Decylphosphat Isotridecylphosphat mit einer Säurezahl von 220 mg KOH/g d.h. das in etwa aus gleichen Anteilen Monoester und Diester besteht, eingesetzt wurde, das unter dem Namen "Servoxyl VPDZ 100" bei der Elementis GmbH, D-Köln erhältlich ist.

### Vergleichsbeispiel V1

100 Teile eines mit α,ω-hydroxyterminierten Polydimethylsiloxans mit einer Viskosität von 60 mPas wurden in einem Becher vorgelegt. Mit einem Rotor-Stator-Homogenisator (Ultra-Turrax, Umfangsgeschwindigkeit 16 m/s) wurden 10 Teile eines Octyl-/Decylphosphats mit einer Säurezahl von 295 mg KOH/g (erhältlich unter dem Namen "Crodafos 810 A" bei der Croda GmbH, D-Nettetal), 14 Teile eines ethoxylierten Laurylalkohols der Formel C₁₂H₂₃-O-(CH₂CH₂O)₂₃-H (erhältlich unter dem Namen "Brij 35" bei der Croda GmbH, D-Nettetal) und 10 Teile Wasser zugegeben und 10 min homogenisiert. Die entstehende gelartige Paste (Fließgrenze 1940 Pa) mit einer Partikelgröße von kleiner als 200 nm hatte eine Temperatur von 23 °C. Die Paste wurde innerhalb von 10 min mit 100 Teilen Wasser verdünnt und bei 5 °C gelagert. Diese Emulsion hatte einen pH-Wert von 1,6. Nach 72 Stunden wird die Emulsion mit Triethanolamin auf einen pH-Wert von 7 eingestellt, und es wurden 0,18 Teile Konservierungsmittel auf der Basis von Methylisothiazolinon in Kombination mit Ethylhexylglycerin (erhältlich unter der Bezeichnung "Euxyl K220" bei Schülke&Mayr GmbH, Norderstedt) zugesetzt.

### Vergleichsbeispiel V2

Das Vergleichsbeispiel V1 wurde wiederholt. Allerdings wurde das Octyl-/Decylphosphat sofort mit Triethanolamin neutralisiert und nach Abkühlen der Emulsion auf 5 °C wieder mit Phosphorsäure angesäuert.
Nach Ablauf der Kondensationszeit wurde die Emulsion erneut mit Triethanolamin neutralisiert. Danach trennte sich die Emulsion aufgrund des zu hohen Salzgehaltes innerhalb weniger Stunden und konnte nicht weiter verwendet werden.

### Vergleichsbeispiel V3

Beispiel 1 wurde wiederholt, wobei aber der Phosphorsäureester bei einer Temperatur von 40 °C zugesetzt und die Emulsion 48 Stunden bei Raumtemperatur gelagert wurde.

### Vergleichsbeispiel V4

Beispiel 1 wurde wiederholt aber anstelle des Phosphorsäureesters werden 3 % einer Alkylbenzolsulfonsäure (erhältlich unter dem Namen Marlon AS 3 Säure bei der SASOL AG D-Marl) eingesetzt.

**Tabelle 1:**

| Beispiel | Teilchengröße D(50) in nm | Ölviskosität in mm²/s | D₄ in ppm¹⁾ | D₄ in Gew. -%²⁾ |
|---|---|---|---|---|
| 1 | 175 | 1190000 | 228 | 0,054 |
| 2 | 82 | 930000 | 380 | 0,090 |
| 3 | 112 | 1810000 | 490 | 0,117 |
| 4 | 87 | 1490000 | 330 | 0,079 |
| 5 | 94 | 669000 | 270 | 0,064 |
| V1 | 97 | 1780000 | 1100 | 0,262 |
| V2 | Emulsion trennte sich nach der Neutralisation | | | |
| V3 | 120 | 548000 | 1550 | 0,369 |
| V4 | 142 | 1140000 | 2260 | 0,538 |

| | | | | |
|---|---|---|---|---|
| ¹⁾ bezogen auf die Emulsion, ²⁾ bezogen auf Siloxan | | | | |

Überraschenderweise führt die nachträgliche Zugabe des sauren Phosphates nicht zu einer langsameren Kondensationsreaktion.

Die Bildung an D₄ ist jedoch im Vergleich zum Stand der Technik deutlich zurückgedrängt. Die Stabilität der Emulsion bei der, wie es in EP-A 1 072 629 vorgeschlagen wird, der saure anionische Emulgator vor der Emulsionsherstellung neutralisiert und dann wieder durch Zugabe von Säure aktiviert wird, war ungenügend. Das Vergleichsbeispiel 3 zeigt, dass ein dem erfindungsgemäßen Verfahren analoger Prozess mit herkömmlichen Emulgatoren zu einer wesentlich stärkeren Bildung von D₄ führt.

### Beispiel 6

### Herstellung einer grobteiligen Emulsion

100 Teile eines mit α,ω-hydroxyterminierten Polydimethylsiloxans mit einer Viskosität von 100 mPas wurden bei 25 °C in einem Becher vorgelegt. Mit einem Flächenrührer wurden 14 Teile eines ethoxylierten Laurylalkohols der Formel C₁₂H₂₃-O-(CH₂CH₂O)₂₃-H (erhältlich unter dem Namen "Brij 35" bei der Croda GmbH, D-Nettetal) gelöst in 20 Teilen Wasser zugegeben, und es wurde 10 min gerührt. Weitere 40 Teile Wasser wurden schrittweise zugesetzt.
Anschließend wurden bei einer Temperatur von 5 °C 6 Teile eines Octyl-/Decylphosphats mit einer Säurezahl von 295 mg KOH/g (erhältlich unter dem Namen "Crodafos 810 A" bei der Croda GmbH, D-Nettetal) eingerührt. Der pH-Wert betrug 1,6. Die Emulsion wurde 48 Stunden bei 5 °C gelagert und anschließend mit Triethanolamin auf einen pH-Wert von 7 eingestellt, und es wurden 0,18 Teile Konservierungsmittel auf der Basis von Methylisothiazolinon in Kombination mit Ethylhexylglycerin (erhältlich unter der Bezeichnung "Euxyl K220" bei Schülke&Mayr GmbH, Norderstedt) zugesetzt. Die Ergebnisse der Prüfung sind in Tabelle 2 zusammengestellt.

### Vergleichsbeipiel 5

Beispiel 6 wurde wiederholt, aber anstelle des Phosphorsäureesters wurden 3 % einer Alkylbenzolsulfonsäure (erhältlich unter dem Namen Marlon AS 3 Säure bei der SASOL AG D-Marl) eingesetzt. Die Emulsion wurde 72 Stunden bei 5 °C gelagert und dann neutralisiert.

**Tabelle 2:**

| Beispiel | Teilchengröße D(50) in µm | Ölviskosität in mm²/s | D₄ in ppm¹⁾ | D₄ in Gew.-%²⁾ |
|---|---|---|---|---|
| 6 | 18 | 1820000 | 400 | 0,070 |
| V5 | 31 | 20000 | 2810 | 0,493 |

| | | | | |
|---|---|---|---|---|
| ¹⁾ bezogen auf die Emulsion, ²⁾ bezogen auf Siloxan Überraschenderweise zeigt die nach dem erfindungsgemäßen Verfahren hergestellte grobteilige Emulsion eine effiziente Polykondensation und damit hohe Ölviskosität bei einem sehr niedrigen Gehalt an D₄, während das Vergleichsbeispiel bereits einen hohen Anteil an D₄ enthält, ohne dass eine sehr hohe Ölviskosität erreicht worden wäre. | | | | |

### Beispiel 7

Ein Shampoo wird wie folgt formuliert (Die Komponenten werden entsprechend der INCI-Nomenklatur bezeichnet):

0,2 Teile Guar Hydroxypropyltrimoniumchlorid (erhältlich unter dem Namen N-Hance^{®} 3000 bei der Hercules Inc.) werden in 11,92 Teilen Wasser dispergiert. 71,7 Teile Sodium Laureth Sulfat (erhältlich unter dem Namen Genapol LRO 26,5 % bei der Clariant GmbH) werden langsam eingerührt und die Mischung wird auf 75 °C erwärmt. Dabei werden 0,3 Teile PEG-150 Distearate (erhältlich unter dem Namen Emulgin EO 33 bei der Cognis Deutschland GmbH) bei Erreichen von 50 °C zugegeben und wenn 65 °C erreicht sind 1,2 Teile Glycol Distearate (erhältlich unter dem Namen Genapol PMS bei der Clariant GmbH). Die Mischung wird gemischt bis 75 °C erreicht sind. Dann wird die Mischung abgekühlt. Wenn 35 °C erreicht sind werden 0,6 Teile Konservierer Kathon CG (erhältlich bei der Acima Chemical Industries Ltd.Inc. CH-9471 Buchs) und 4 Teile der Emulsion von Beispiel 2 zugegeben und 5 Minuten gerührt. Abschließend werden 10,06 Teile Cocamidopropyl Betaine (erhältlich unter dem Namen Genagen CAB 30 % bei der Clariant GmbH) und 0,56 Teile Natriumchlorid zugegeben und jeweils 10 Minuten gerührt. Dieses Shampoo verbessert sowohl die Trocken- und die Nasskämmbarkeit als auch das Griffgefühl im nassen und trockenen Haar.

## Patentansprüche

1. Verfahren zur Herstellung von Emulsionen von Organopolysiloxanen, **dadurch gekennzeichnet, dass**
in einem ersten Schritt
(a) Polyorganosiloxane enthaltend Einheiten der allgemeinen Formel
R²ₐ(R¹O)_{b}SiO_{(4-a-b)/2} (I),
worin
R² gleich oder verschieden sein kann und einen einwertigen, gegebenenfalls substituierten Kohlenwasserstoffrest mit 1 bis 30 Kohlenstoffatomen oder Wasserstoffatom bedeutet,
R¹ gleich oder verschieden sein kann und Wasserstoffatom oder einen einwertigen, gegebenenfalls substituierten Kohlenwasserstoffrest bedeutet,
a 0, 1, 2 oder 3 ist und
b 0, 1, 2 oder 3 ist,
mit der Maßgabe, dass die Summe a+b kleiner oder gleich 3 ist und die Organopolysiloxane 5 bis 500 Einheiten der Formel (I) enthalten und in mindestens einer Einheit b verschieden 0 ist,
(b) nichtionogene Emulgatoren,
(c) Wasser und gegebenenfalls
(d) weitere Stoffe durch Rühren und/oder Homogenisieren vermischt werden,
in einem zweiten Schritt
zu der im ersten Schritt erhaltenen Emulsion
(e) saure oberflächenaktive Verbindungen der allgemeinen Formel
(RO)ₙP(O)(OH)₍₃₋ₙ₎ (II),
worin
R gleich oder verschieden sein kann und einwertige Kohlenwasserstoffreste mit 4 bis 30 Kohlenstoffatomen bedeutet und n 1 oder 2 ist,
bei einer Temperatur unter 30 °C, wozu die im ersten Schritt erhaltene Emulsion gegebenenfalls abgekühlt werden muss, zugegeben und die Polyorganosiloxane (a) kondensieren gelassen werden bis die gewünschte Viskosität erreicht ist und
in einem dritten Schritt
die im zweiten Schritt erhaltene Mischung mit Base (f) versetzt wird, so dass der pH-Wert der Emulsion größer als 5 ist, und gegebenenfalls weiteres Wasser (c) und/oder weitere Stoffe (d) zugegeben werden.

2. Verfahren nach Anspruch 1, **dadurch gekennzeichnet, dass** es sich bei den Siloxanen (a) um solche der Formel
HO [SiR²₂O]_{c}-H (III)
handelt, wobei R² eine der obengenannten Bedeutungen hat und c einen Wert von 5 bis 500 besitzt.

3. Verfahren nach Anspruch 1 oder 2, **dadurch gekennzeichnet, dass** als Emulgatoren (b) solche eingesetzt werden, die ausgewählt werden aus
(b1) ethoxylierten Triglyceriden mit 40 bis 400 Ethylenglycolgrupppen,
(b2) ethoxylierten Sorbitanestern von Fettsäuren mit 12 bis 18 Kohlenstoffatomen und 10 bis 40 Ethylenglycolgruppen,
(b3) Verbindungen der Formel
R³-O-(CH₂CH₂O)ₘ-H (IV)
und
(b4) Verbindungen der Formel
R⁴CH₂C(O)-O-(CH₂CH₂O)ₚ-H (V),
worin
R³ einen Alkylrest mit 10 bis 30 Kohlenstoffatomen bedeutet,
R⁴ einen Alkylrest mit 10 bis 30 Kohlenstoffatomen bedeutet,
m einen Wert von 5 bis 100 darstellt und
p einen Wert von 5 bis 100 annimmt.

4. Verfahren nach einem oder mehreren der Ansprüche 1 bis 3, **dadurch gekennzeichnet, dass** es sich bei Verbindungen der Formel (II) um Mono-n-octylphosphat, Di-n-octylphosphat, Mono-n-decylphosphat, n-Octyl-n-Decylphosphat und Di-n-decylphosphat handelt.

5. Verfahren nach einem oder mehreren der Ansprüche 1 bis 4, **dadurch gekennzeichnet, dass** der pH-Wert der im ersten Schritt erhaltenen Emulsion mehr als 3,5 beträgt.

6. Verfahren nach einem oder mehreren der Ansprüche 1 bis 5, **dadurch gekennzeichnet, dass** der zweite Schritt bei einer Temperatur unter 20 °C durchgeführt wird.

7. Verfahren nach einem oder mehreren der Ansprüche 1 bis 6, **dadurch gekennzeichnet, dass** der pH-Wert der im zweiten Schritt erhaltenen Emulsion weniger als 2,5 beträgt.

8. Verfahren nach einem oder mehreren der Ansprüche 1 bis 7, **dadurch gekennzeichnet, dass** es sich bei den Basen (f), die im dritten Schritt eingesetzt werden, um Alkali- bzw. Erdalkalihydroxide, Ammoniak und Amine handelt.

9. Verfahren nach einem oder mehreren der Ansprüche 1 bis 8, **dadurch gekennzeichnet, dass** der pH-Wert der Emulsion nach Durchführung des dritten Schritts 5 bis 10 beträgt.

10. Verfahren nach einem oder mehreren der Ansprüche 1 bis 9, **dadurch gekennzeichnet, dass** die hergestellten Emulsionen weniger als 0,2 Gew.-% Octaorganylcyclotetrasiloxan enthalten, bezogen auf eingesetzte Komponente (a).

## Claims

1. Method for producing emulsions of organopolysiloxanes, **characterized in that**
in a first step
(a) polyorganosiloxanes containing units of the general formula
R²ₐ(R¹O)_{b}SiO_{(4-a-b)/2} (I),
in which
R² can be identical or different and is a monovalent, optionally substituted hydrocarbon radical having 1 to 30 carbon atoms or hydrogen atom,
R¹ can be identical or different and is hydrogen atom or a monovalent, optionally substituted hydrocarbon radical,
a is 0 , 1, 2 or 3 and
b is 0, 1, 2 or 3,
with the proviso that the sum a + b is less than or equal to 3 and the organopolysiloxanes contain 5 to 500 units of the formula (I) and in at least one unit b is other than 0,
(b) nonionogenic emulsifiers,
(c) water and
optionally
(d) further substances
are mixed by stirring and/or homogenization,
in a second step
(e) acidic surface-active compounds of the general formula
(RO)ₙP(O)(OH)₍₃₋ₙ₎ (II),
in which
R can be identical or different and is monovalent hydrocarbon radicals having 4 to 30 carbon atoms and n is 1 or 2,
are added to the emulsion obtained in the first step at a temperature below 30°C, to which end the emulsion obtained in the first step must be optionally cooled, and the polyorganosiloxanes (a) are left to condense until the desired viscosity is reached and
in a third step
the mixture obtained in the second step is admixed with base (f), such that the pH of the emulsion is greater than 5, and optionally further water (c) and/or further substances (d) are added.

2. Method according to Claim 1, **characterized in that** the siloxanes (a) are those of the formula
HO[SiR²₂O]_{c}-H (III)
where R² has one of the aforementioned meanings and c has a value from 5 to 500.

3. Method according to Claim 1 or 2, **characterized in that** the emulsifiers (b) used are those which are selected from
(b1) ethoxylated triglycerides having 40 to 400 ethylene glycol groups,
(b2) ethoxylated sorbitan esters of fatty acids having 12 to 18 carbon atoms and 10 to 40 ethylene glycol groups,
(b3) compounds of the formula
R³-O-(CH₂CH₂O)ₘ-H (IV)
and
(b4) compounds of the formula
R⁴CH₂C(O)-O-(CH₂CH₂O)ₚ-H (V),
in which
R³ is an alkyl radical having 10 to 30 carbon atoms,
R⁴ is an alkyl radical having 10 to 30 carbon atoms,
m is a value from 5 to 100 and
p assumes a value from 5 to 100.

4. Method according to one or more of Claims 1 to 3, **characterized in that** compounds of the formula (II) are mono-n-octyl phosphate, di-n-octyl phosphate, mono-n-decyl phosphate, n-octyl-n-decyl phosphate and di-n-decyl phosphate.

5. Method according to one or more of Claims 1 to 4, **characterized in that** the pH of the emulsion obtained in the first step is more than 3.5.

6. Method according to one or more of Claims 1 to 5, **characterized in that** the second step is carried out at a temperature below 20°C.

7. Method according to one or more of Claims 1 to 6, **characterized in that** the pH of the emulsion obtained in the second step is less than 2.5.

8. Method according to one or more of Claims 1 to 7, **characterized in that** the bases (f) which are used in the third step are alkali metal or alkaline earth metal hydroxides, ammonia and amines.

9. Method according to one or more of Claims 1 to 8, **characterized in that** the pH of the emulsion after carrying out the third step is 5 to 10.

10. Method according to one or more of Claims 1 to 9, **characterized in that** the emulsions produced contain less than 0.2% by weight of octaorganylcyclo-tetrasiloxane, based on component (a) used.

## Revendications

1. Procédé de fabrication d'émulsions d'organopolysiloxanes, **caractérisé en ce que** lors d'une première étape,
(a) des polyorganosiloxanes contenant des unités de formule générale
R²ₐ(R¹O)_{b}SiO_{(4-a-b)/2} (I)
dans laquelle
les R² peuvent être identiques ou différents et signifient un radical hydrocarboné monovalent, éventuellement substitué, de 1 à 30 atomes de carbone, ou un atome d'hydrogène,
les R¹ peuvent être identiques ou différents et signifient un atome d'hydrogène ou un radical hydrocarboné monovalent, éventuellement substitué,
a représente 0, 1, 2 ou 3, et
b représente 0, 1, 2 ou 3,
à condition que la somme a+b soit inférieure ou égale à 3 et que les organopolysiloxanes contiennent 5 à 500 unités de formule (I) et que b soit différent de 0 dans au moins une unité,
(b) des émulsifiants non ionogènes,
(c) de l'eau et
éventuellement
(d) des substances supplémentaires,
sont mélangés par agitation et/ou homogénéisation,
lors d'une deuxième étape,
(e) des composés tensioactifs acides de formule générale
(RO)ₙP(O)(OH)₍₃₋ₙ₎ (II)
dans laquelle
les R peuvent être identiques ou différents et signifient des radicaux hydrocarbonés monovalents de 4 à 30 atomes de carbone, et
n représente 1 ou 2,
sont ajoutés à l'émulsion obtenue lors de la première étape à une température inférieure à 30 °C, l'émulsion obtenue lors de la première étape devant éventuellement être refroidie, et les polyorganosiloxanes (a) sont laissés se condenser jusqu'à atteindre la viscosité souhaitée, et
lors d'une troisième étape,
le mélange obtenu lors de la deuxième étape est mélangé avec une base (f), de sorte que le pH de l'émulsion soit supérieur à 5, et éventuellement de l'eau supplémentaire (c) et/ou des substances supplémentaires (d) sont ajoutées.

2. Procédé selon la revendication 1, **caractérisé en ce que** les siloxanes (a) sont de formule
HO[SiR²₂O]_{c}-H (III)
dans laquelle R² a une des significations susmentionnées et c a une valeur de 5 à 500.

3. Procédé selon la revendication 1 ou 2, **caractérisé en ce que** les émulsifiants (b) utilisés sont choisis parmi
(b1) les triglycérides éthoxylés contenant 40 à 400 groupes éthylène glycol,
(b2) les esters de sorbitane éthoxylés d'acides gras contenant 12 à 18 atomes de carbone et 10 à 40 groupes éthylène glycol,
(b3) les composés de formule
R³-O-(CH₂CH₂O)ₘ-H (IV)
et
(b4) les composés de formule
R⁴CH₂C(O)-O-(CH₂CH₂O)ₚ-H (V)
dans lesquelles
R³ signifie un radical alkyle de 10 à 30 atomes de carbone,
R⁴ signifie un radical alkyle de 10 à 30 atomes de carbone,
m a une valeur de 5 à 100, et
p a une valeur de 5 à 100.

4. Procédé selon une ou plusieurs des revendications 1 à 3, **caractérisé en ce que** les composés de formule (II) sont le phosphate de mono-n-octyle, le phosphate de di-n-octyle, le phosphate de mono-n-décyle, le phosphate de n-octyle-n-décyle et le phosphate de di-n-décyle.

5. Procédé selon une ou plusieurs des revendications 1 à 4, **caractérisé en ce que** le pH de l'émulsion obtenue lors de la première étape est supérieur à 3,5.

6. Procédé selon une ou plusieurs des revendications 1 à 5, **caractérisé en ce que** la deuxième étape est réalisée à une température inférieure à 20°C.

7. Procédé selon une ou plusieurs des revendications 1 à 6, **caractérisé en ce que** le pH de l'émulsion obtenue lors de la deuxième étape est inférieur à 2,5.

8. Procédé selon une ou plusieurs des revendications 1 à 7, **caractérisé en ce que** les bases (f) utilisées lors de la troisième étape sont des hydroxydes alcalins ou alcalino-terreux, de l'ammoniac et des amines.

9. Procédé selon une ou plusieurs des revendications 1 à 8, **caractérisé en ce que** le pH de l'émulsion après la réalisation de la troisième étape est de 5 à 10.

10. Procédé selon une ou plusieurs des revendications 1 à 9, **caractérisé en ce que** les émulsions fabriquées contiennent moins de 0,2 % en poids d'octaorganylcyclotétrasiloxane, par rapport au composant (a) utilisé.
